# EUROPEAN PATENT SPECIFICATION

(11) **EP 0 777 477 B1**
(45) Date of publication and mention of the grant of the patent: **13.11.2002**
(21) Application number: 95928574.3
(22) Date of filing: 16.08.1995
(51) Int. Cl.: A61K 31/485, A61K 9/20

(54) **IMPROVED PHARMACEUTICAL FORMULATIONS CONTAINING IBUPROFEN AND CODEINE**
VERBESSERTE,IBUPROFEN UND CODEIN ENTHALTENDE,PHARMAZEUTISCHE ZUSAMMENSETZUNGEN
FORMULATIONS PHARMACEUTIQUES AMELIOREES CONTENANT DE L'IBUPROFENE ET DE LA CODEINE

(30) Priority: 23.08.1994 GB 9417041; 04.10.1994 GB 9419921
(43) Date of publication of application: 11.06.1997
(73) Proprietor: SmithKline Beecham plc, Brentford, Middlesex TW8 9GS (GB)
(72) Inventor: HOLMES, Brian Sterling Health Europe Sanofi-Winth, Fawdon Newcastle-upon-Tyne NE3 3TT (GB)
(74) Representative: White, Susan Mary
(86) International application number: GB9501947
(87) International publication number: WO96005834

(56) References cited:
- EP-A- 0 388 125
- EP-A- 0 535 841
- US-A- 4 571 400
- SUCKER H.; FUCHS P.; SPEISER P. 'PHARMAZEUTISCHE TECHNOLOGIE' 1991 , STUTTGART, THIEME VERLAG.; DE see page 259, column 2, paragraph 3

## Description

The present invention relates to improved pharmaceutical formulations comprising ibuprofen and codeine (as active ingredients), methods for preparing such formulations and their use in therapy.

Ibuprofen, the chemical name for which 2-(4-isobutylphenyl)propionic acid, is well-known as a non-steroidal anti-inflammatory drug (or NSAID). It has anti-inflammatory, antipyretic and analgesic action and has thus been used, either in the form of the acid itself or as a pharmaceutically acceptable salt thereof, such as the sodium salt, in the treatment or prophylaxis of pain and inflammation such as in rheumatoid arthritis, headache, neuralgia, dysmenorrhoea, for reducing platelet adhesiveness, for dental pain and the like.

Codeine, the chemical name for which is 7,8-didehydro-4,5-epoxy-3-methoxy-17-methyl-morphinan-6-ol, is also well-known and is a narcotic analgesic used, usually in the form of a pharmaceutically acceptable salt such as acetate, hydrobromide, hydrochloride, salicylate, sulphate or, preferably, the phosphate, often in hydrated or partially hydrated form such as the hemihydrate, in the treatment or prophylaxis of pain, particularly more severe pain.

The combination of ibuprofen and codeine has been suggested as having a particularly beneficial effect as an analgesic. For example, Cooper et al in Clin. Pharm. and Ther. 27(2), 249 (1980) reported the advantageous effects of administering the combination to patients having moderate-to-severe pain following dental impaction surgery. European patent specification no. 388 125 discloses the use of the combination in the treatment of acute pain such as headaches and dental pain.

European patent specification no. 535 841 discloses the use of the combination in the treatment of the pain of chronic medical conditions such as osteoarthritis, rheumatoid arthritis, ankylosing spondylitis, seroarthropathies, bursitis, capsulitis of the shoulder, tendinitis, tenosynovitis and cancer.

However, it has, for some time, been recognised that the storage properties of pharmaceutical formulations containing a combination of ibuprofen with codeine are unsatisfactory. This is especially so in the case of the most popular method of administration of the combination, viz. in the form of a tablet. Over time and with increasing temperature, traditional formulations of ibuprofen plus codeine can be seen to discolour and start to disintegrate by cracking or expanding. Thus, the problem to be addressed is how to produce 'white' tablets (i.e. wherein the ingredients have good colour stability and do not discolour or yellow over time or temperature) and which equally do not disintegrate on storage. The ingredients are preferably, for formulation purposes, also directly compressible.

This same problem is addressed in European patent specification no. 159 852, but from the point-of-view of avoiding intimate physical contact between the active ingredients during the wet granulation process for making tablets. The solution put forward was to use an alternative process, namely: blending one of the active ingredients with binder and filler; wet-granulating the mixture so produced in the presence of a solvent; drying the granulated mixture; sizing the granulated mixture; and blending the mixture with one or more other pharmacologically active components. However, the disadvantages associated with such a multi-step process are self-evident.

An alternative solution to the problem is disclosed in European patent specification no. 220 805, in particular the difficulties of poor crushing strength and long disintegration times associated with ibuprofen plus codeine tablets. The solution proposed therein was to form a multiphase tablet wherein the, for example codeine, is free from the, for example, ibuprofen, stearic acid and stearate salt, and the, for example, ibuprofen is likewise free from the, for example, codeine, stearic acid and stearate salt, and further wherein both the codeine phase and the ibuprofen phase contain at least one self-lubricating compression aid (such as microcrystalline cellulose or a free flowing, directly compressible starch). However, even this alternative solution presents its own problems by being of multiphase composition. The aforementioned European patent specification no. 535 841 also discloses bilayer tablets.

However, disclosures of monophasic formulations of ibuprofen plus codeine teach that stearic acid and/or stearate are necessary to provide a satisfactory formulation. For example, European patent specification no. 68 838, although not relating to ibuprofen/codeine specifically, discloses only tableted combinations of flurbiprofen with a narcotic analgesic which include magnesium stearate. The aforementioned European patent specification no. 388 125 discloses only tablets of ibuprofen plus codeine having stearic acid in the formulation. The same problem is also addressed in European patent specification no. 274 845 where the solution disclosed is to incorporate insoluble salts of carboxymethylcellulose in a solid composition containing ibuprofen plus codeine. However, even this approach teaches the inclusion of stearic acid or magnesium stearate as lubricant, particularly stearic acid.

It seemed, therefore, as if the only way to avoid stearic acid/stearate in the formulation, which was believed to give rise to or exacerbate the storage problems, was to employ a multiphase formulation. However, we have surprisingly found that a formulation can be prepared without the use of stearic acid or stearate and which is monophasic, directly compressible, and which overcomes the stability and storage problems previously associated with ibuprofen plus codeine formulations by employing a hydrogenated vegetable oil as lubricant.

Thus, the present invention provides a pharmaceutical formulation comprising ibuprofen and codeine (or a pharmaceutically-acceptable salt of either of them) in association with a pharmaceutically acceptable carrier wherein said carrier comprises a lubricant, a disintegrant and a diluent characterised in that the lubricant is substantially free from stearic acid and stearate ions and is selected from hydrogenated vegetable oils.

Preferably, the lubricant and, more preferably, the formulation is also substantially free of metal ions such as magnesium or other trace impurities such as aluminium and iron. By "substantially free" in this context is meant that metal ions may be present in the parts per million range (but preferably less than about 10ppm) but preferably not in the parts per hundred or percentage range.

A preferred hydrogenated vegetable oil is selected from hydrogenated soybean oil and hydrogenated castor oils, and mixtures thereof such as glycerol esters. Examples of such preferred oils are glycerol esters of C₁₆-C₂₀ and glycerol tris 12-hydroxy stearate. One such preferred oil is sold under the name 'Sterotex K' available from Karlshamns, Hull HU1 0QA, UK. Also suitable are Castorwax, Castorwax MP70, Castorwax MP80, Cenwachs G, Cerit CH, Opalwax, Ceroxin and Cutina.

Especially surprising is that, in formulations according to the present invention, disintegrants such as cross-linked polyvinylpyrrolidone (PVP), croscarmellose sodium and sodium starch glycolate can all be used satisfactorily in such formulations. In particular, croscarmellose sodium and especially sodium starch glycolate or mixtures thereof are preferred disintegrants for use in these formulations. Our surprise is due in part to the express teaching in the aforementioned European patent specification no. 274 845 that formulations "containing effective levels of the listed disintegrants were stored at 50°C and observed regularly for discolouration and for swelling or splitting. Only compositions containing calcium carboxymethylcellulose gave satisfactory results and all other formulations showed unsatisfactory storage properties due to one or more of discolouration, swelling or splitting within a 12 week period". On the contrary, we have found that, when a hydrogenated vegetable oil is included as lubricant, satisfactory storage results are obtained in the presence of the listed disintegrants in terms of the tablets remaining 'white' and stable over time and temperature (e.g. for three months at 40°C).

The present invention therefore further provides a formulation as hereinbefore defined wherein the disintegrant is preferably selected from croscarmellose sodium; a starch glycolate salt such as sodium starch glycolate (available from Tunnel Avebe Starches Ltd.); and polyvinylpyrrolidone (PVP) such as cross-linked PVP; and mixtures thereof. More preferably, sodium starch glycolate and/or up to 20mg per 660mg formulation of croscarmellose sodium are the disintegrant(s).

Preferably, the formulations further comprise a diluent which is readily compressible such as one selected from lactose and cellulose, or mixtures thereof, preferably β-lactose monohydrate and/or cellulose, more preferably 72-76% lactose monohydrate plus 23-27% cellulose (substantially 3:1), optionally in the presence of 3-5% water based on weight of lactose and cellulose, for example, Cellactose [Registered Trade Mark] such as Cellactose 80 which is available from Meggle in Germany; and optionally microcrystalline cellulose such as microcrystalline cellulose PH102 (available as Avicel from F.M.C.) and/or carboxymethyl cellulose. Cellactose is the especially preferred diluent.

Other ingredients may also be added to the formulation such as are conventional in the art (but other than stearic acid or stearates) such as a flow enhancer or further lubricant, for example a colloidal silica such as colloidal silicon dioxide available, for example as Aerosil from Wacker-Chemie GmbH/Degussa in Germany, preferably up to about 3mg per 200mg ibuprofen. Other ingredients include, but are not limited to, binders or compression aids such as pregelatinised maize starch, hydroxypropylmethyl cellulose (available from Shinetsu/Dow), microcrystalline cellulose, polyvinyl-pyrrolidone, gelatin and gums; diluents or fillers such as lactose, sodium chloride, sorbitol, mannitol, microcrystalline cellulose, calcium phosphates and calcium sulphate; additional lubricants such as polyethylene glycols (for example, PEG 400 and PEG 6000) (but excluding as hereinbefore mentioned, stearic acid or its salts); surfactants or wetting agents such as sodium lauryl sulphate preferably about 2mg per 200mg ibuprofen and the commercially-available Tween 80 (Trade Mark); oils; fats; waxes; and colouring and flavouring agents.

Preferred other ingredients are selected from colloidal silicon dioxide, maize starch, hydroxypropylmethyl cellulose, microcrystalline cellulose, polyvinyl-pyrrolidone, lactose, sorbitol, mannitol, polyethylene glycols, oils, fats, waxes, and colouring and flavouring agents.

Other active ingredient(s) may also be present such as caffeine or other active ingredients suitable for use in association with ibuprofen and codeine such as decongestants, antihistamines and antitussives, for example pseudoephedrine, diphenhydramine and the like.

As can be appreciated from the foregoing, although various forms of the formulation can be adopted, solid dose forms are preferred and the most preferred form is that of a monophasic tablet. Such tablets are preferably comprised of a tablet core according to the invention and a coating thereover which coating preferably comprises a film of a film-forming polymer such as hydroxypropylmethyl cellulose, a plasticiser such as a polyethylene glycol (such as PEG 400) and, optionally, a colouring dispersion. It is preferred that colour be applied to the tablets by coloured film-coating rather than incorporating a colouring agent directly into the core granules before compression. Preferably, the film coating comprises about 1% by weight of the total formulation. If desired, the tablets can then be printed using a standard food grade ink. Alternatively, they can be embossed.

The ibuprofen and codeine may be present, for example, in the form of an aforementioned pharmaceutically acceptable salt in any therapeutically-effective amount. For example, ibuprofen may be present in the range 50-2400mg, e.g. 300 to 1200mg, preferably 200-800mg. Especially preferred unit dose formulations contain 200mg, 300mg or 600mg ibuprofen, especially 200mg. The codeine may be present in the range of 10 to 300 mg, preferably 10 to 100mg; for example, as the phosphate hemihydrate, 12 to 22.5mg, such as about 13.5mg.

A particularly preferred formulation comprises about 200mg ibuprofen plus about 13.57mg codeine phosphate hemihydrate (corresponding to 10mg codeine) within a tablet core of about 660mg, such as 663mg. Optionally, up to 65mg, preferably about 30mg caffeine may also be present.

Preferably, formulations are provided such that a maximum human dose regime comprises two unit doses, four times per day.

All amounts of ingredients or weights of components given herein are subject to the usual pharmaceutically acceptable tolerances. Such tolerances are usually accepted to be ±5%. For example, the preferred amounts of ibuprofen or codeine range from 95%-105% of the figures specified.

The formulations may be prepared by any method known in the art. For example, monophasic tablets may be prepared by the direct compression method wherein all the ingredients of the tablet core are sieved together, blended and then compressed. This direct compression method avoids the need for pretreatment of the powdered ingredients such as by wet granulation followed by drying. Preferably, compression results in capsule-shaped tablets or 'caplets'.

In a broader aspect, the present invention therefore provides a method of preparing a formulation according thereto, which method comprises bringing ibuprofen and codeine (or a salt of either of them) into association with a hydrogenated vegetable oil. Preferably, the amount of moisture present in the ingredients and during processing is kept to a minimum; more preferably, the moisture content is less than about 5% w/w of the formulation.

Once prepared, tablets such as 'caplets' according to the invention are packed into a suitable container such as a glass or plastics bottle or, preferably, a so-called 'blister' pack such as one comprising polyvinyl chloride 'blisters' heat-sealed with aluminium foil. Particularly for providing a so-called 'patient pack', the container is then packaged in a carton which further contains the patient instruction leaflet giving information regarding use and dosage of the formulation.

As will be understood by those skilled in the art, the present invention further provides the use of a formulation according thereto in therapy, in particular in the treatment or prophylaxis of any of the conditions hereinbefore described, especially mild to moderate or severe pain. The present invention hence provides the use of a formulation according there to in the preparation of a medicament for use in a method of treating such a condition, which method comprises the administration to a patient in need thereof of an effective amount of a pharmaceutical formulation according to the invention.

The present invention will now be illustrated by the following examples, although other methods of putting it into effect will be evident to those skilled in the art.

### EXAMPLE 1 - Tablets

| Tablet Core | mg/tablet |
|---|---|
| Ibuprofen | 200.00 |
| Codeine phosphate hemihydrate | 13.57 |
| Hydrogenated vegetable oil | 5.00 |
| Cellactose | q.s. to 660mg |
| Aerosil | 3.00 |
| Disintegrant^{x} | 55.00 |

| | |
|---|---|
| ^{x}Sodium starch glycolate and/or croscarmellose sodium (Acdi-sol). | |

The ingredients are sieved together, blended and compressed together, using the direct compression method.

### EXAMPLE 2 - Tablets

| Tablet Core | mg/tablet |
|---|---|
| Ibuprofen | 200.00 |
| Codeine phosphate hemihydrate | 13.57 |
| Cellactose | 200.00 |
| Microcrystalline cellulose | 183.43 |
| Hydrogenated vegetable oil | 5.00 |
| Sodium starch glycolate | 55.00 |
| Aerosil | 3.00 |

The tablets are prepared according to the method of Example 1.

### EXAMPLE 3 - Film-Coated Tablets

| Tablet Core | mg/tablet |
|---|---|
| Ibuprofen | 200.00 |
| Codeine phosphate hemihydrate | 13.57 |
| Microcrystalline cellulose | 182.93 |
| Hydrogenated vegetable oil | 5.00 |
| Sodium starch glycolate | 55.00 |
| Aerosil (colloidal silicon dioxide) | 0.50 |
| Cellactose | 200.00 |

The tablet core is prepared by the method of Example 1. Thereafter it is coated with a film-coating comprising hydroxypropylmethyl cellulose, PEG 400 and a suitable colouring dispersion in the following quantities:

| Film Coating A | |
|---|---|
| Hydroxypropylmethyl cellulose | 6.2846 |
| Polyethylene glycol 400 | 1.2569 |
| Colour dispersion (1) | 2.4584 = 7.3319mg when wet |

| Film Coating B | |
|---|---|
| Hydroxypropylmethyl cellulose | 6.0964 |
| Polyethylene glycol 400 | 1.2193 |
| Colour dispersion (2) | 2.6843 = 7.1126mg when wet |

### EXAMPLE 4 - Film-Coated Tablets

| Tablet Core | mg/tablet |
|---|---|
| Ibuprofen | 200.00 |
| Codeine phosphate hemihydrate | 13.57 |
| Microcrystalline cellulose | 162.93 |
| Hydrogenated vegetable oil | 5.00 |
| Sodium starch glycolate | 55.00 |
| Aerosil | 0.50 |
| Crosscarmellose sodium | 20.00 |
| Cellactose | 200.00 |

The tablet core is prepared by the method of Example 1. Thereafter it is coated with a film-coating comprising hydroxypropylmethyl cellulose, PEG 400 and a suitable colouring dispersion in the following quantities:

| Film Coating A | |
|---|---|
| Hydroxypropylmethyl cellulose | 6.2846 |
| Polyethylene glycol 400 | 1.2569 |
| Colour dispersion (1) | 2.4584 = 7.3319mg when wet |

| Film Coating B | |
|---|---|
| Hydroxypropylmethyl cellulose | 6.0964 |
| Polyethylene glycol 400 | 1.2193 |
| Colour dispersion (2) | 2.6843 = 7.1126mg when wet |

### EXAMPLE 5 - Film-Coated Tablets

| Tablet Core | mg/tablet |
|---|---|
| Ibuprofen | 200.00 |
| Codeine phosphate hemihydrate | 13.57 |
| Hydrogenated vegetable oil | 5.00 |
| Sodium starch glycolate | 55.00 |
| Aerosil | 0.50 |
| Crosscarmellose sodium | 20.00 |
| Cellactose | 362.93 |

The tablet core was prepared according to the method of Example 1, and then film-coated according to the coatings of Examples 4A and 4B.

### EXAMPLE 6 - Caffeine-containing Tablets

| Tablet Core | mg/tablet |
|---|---|
| Ibuprofen | 200.00 |
| Codeine phosphate hemihydrate | 13.57 |
| Cellactose | 355.08 |
| Sterotex K | 5.00 |
| Sodium starch glycolate | 55.00 |
| Caffeine | 30.00 |

The tablet core was prepared according to the method of Example 1. Film-coating according to the method of Examples 4A and 4B can be undertaken to produce film-coated tablets.

### EXAMPLE 7 - Patient Packs

Film-coated tablets as prepared according to Examples 3 to 6 are packed into white, opaque, 200 micron polyvinyl chloride blisters and heat sealed with 20 micron aluminium foil. The blister strips are packed into cardboard cartons to which will be added the patient information leaflet (PIL).

## Claims

1. A pharmaceutical formulation comprising ibuprofen or a pharmaceutically-acceptable salt thereof and codeine or a pharmaceutically-acceptable salt thereof in association with a pharmaceutically acceptable carrier wherein said carrier comprises a lubricant, a disintegrant and a diluent **characterised in that** the lubricant is substantially free from stearic acid and stearate ions, and is selected from hydrogenated vegetable oils.

2. A formulation according to claim 1 wherein the hydrogenated vegetable oil is selected from hydrogenated soybean oil and hydrogenated castor oils, and mixtures thereof.

3. A formulation according to claim 2 wherein the hydrogenated vegetable oil is selected from glycerol esters and mixtures thereof.

4. A formulation according to claim 2 wherein the hydrogenated vegetable oil is selected from C₁₆-C₂₀ glycerol esters and glycerol tris 12-hydroxy stearate.

5. A formulation according to claim 1 wherein the disintegrant is selected from a cross-linked carboxymethyl cellulose salt, a starch glycolate salt, a polyvinylpyrrolidone; and mixtures thereof.

6. A formulation according to claim 5 wherein the disintegrant is selected from cross-linked polyvinylpyrrolidone, croscarmellose sodium and sodium starch glycolate; and mixtures thereof.

7. A formulation according to claim 5 wherein the disintegrant is or includes sodium starch glycolate.

8. A formulation according to claim 1 wherein the diluent comprises lactose, cellulose, or mixtures thereof, and optionally water.

9. A formulation according to claim 8 wherein the diluent is selected from Cellactose (registered trademark), microcrystalline cellulose and carboxymethyl cellulose, and mixtures thereof.

10. A formulation according to claim 8 wherein the diluent comprises lactose and cellulose in a weight ratio of substantially 3:1, and optionally, from 3 to 5% w/w water (based on the weight of lactose and cellulose).

11. A formulation according to any previous claim wherein said carrier further comprises colloidal silicon dioxide.

12. A formulation according to any previous claim comprising a further active ingredient selected from caffeine, decongestants, antihistamines and antitussives; and mixtures thereof.

13. A formulation according to any previous claim comprising 50-2400mg ibuprofen and 10 to 300mg codeine.

14. A formulation according to any previous claim comprising about 200mg ibuprofen and about 13.57mg codeine phosphate hemihydrate; and, optionally, about 30mg caffeine.

15. A formulation according to claim 1 further **characterised in that** the formulation is substantially free (as defined herein) from metal ions.

16. A method of preparing a formulation according to claim 1, which method comprises bringing ibuprofen and codeine (or a pharmaceutically acceptable salt of either of them) into association with hydrogenated vegetable oil.

## Patentansprüche

1. Pharmazeutische Formulierung, die Ibuprofen oder ein pharmazeutisch akzeptables Salz davon und Codein oder ein pharmazeutisch akzeptables Salz davon in Verbindung mit einem pharmazeutisch akzeptablen Träger umfasst, worin der Träger ein Schmiermittel, ein Tablettensprengmittel und ein Verdünnungsmittel umfasst, **dadurch gekennzeichnet, dass** das Schmiermittel im wesentlichen frei von Stearinsäure und Stearationen ist und aus hydrierten pflanzlichen Ölen ausgewählt ist.

2. Formulierung gemäss Anspruch 1, worin das hydrierte pflanzliche Öl aus hydriertem Sojaöl und hydrierten Rizinusölen und Mischungen daraus ausgewählt ist.

3. Formulierung gemäss Anspruch 2, worin das hydrierte pflanzliche Öl aus Glycerinestern und Mischungen daraus ausgewählt ist.

4. Formulierung gemäss Anspruch 2, worin das hydrierte pflanzliche Öl aus C₁₆₋₂₀-Glycerinestern und Glycerintris-12-hydroxystearat ausgewählt ist.

5. Formulierung gemäss Anspruch 1, worin das Tablettensprengmittel aus einem vernetzten Carboxymethylcellulosesalz, einem Stärkeglykolatsalz, einem Polyvinylpyrrolidon und Mischungen daraus ausgewählt ist.

6. Formulierung gemäss Anspruch 5, worin das Tablettensprengmittel aus vernetztem Polyvinylpyrrolidon, Croscarmellose-Natrium und Natrium-Stärkeglykolat und Mischungen daraus ausgewählt ist.

7. Formulierung gemäss Anspruch 5, worin das Tablettensprengmittel Natrium-Stärkeglykolat ist oder dieses einschliesst.

8. Formulierung gemäss Anspruch 1, worin das Verdünnungsmittel Lactose, Cellulose oder Mischungen daraus und gegebenenfalls Wasser umfasst.

9. Formulierung gemäss Anspruch 8, worin das Verdünnungsmittel aus Cellactose (eingetragene Marke), mikrokristalliner Cellulose und Carboxymethylcellulose und Mischungen daraus ausgewählt ist.

10. Formulierung gemäss Anspruch 8, worin das Verdünnungsmittel Lactose und Cellulose in einem Gewichtsverhältnis von im wesentlichen 3:1 und gegebenenfalls 3 bis 5 % G/G Wasser (bezogen auf das Gewicht von Lactose und Cellulose) umfasst.

11. Formulierung gemäss einem der vorhergehenden Ansprüche, worin der Träger ferner kolloidales Siliciumdioxid umfasst.

12. Formulierung gemäss einem der vorhergehenden Ansprüche, die einen weiteren Wirkstoff umfasst, ausgewählt aus Coffein, abschwellenden Mitteln, Antihistaminika und Hustenmitteln und Mischungen daraus.

13. Formulierung gemäss einem der vorhergehenden Ansprüche, die 50 bis 2.400 mg Ibuprofen und 10 bis 300 mg Codein umfasst.

14. Formulierung gemäss einem der vorhergehenden Ansprüche, die ca. 200 mg Ibuprofen und ca. 13,57 mg Codeinphosphathemihydrat; und gegebenenfalls ca. 30 mg Coffein umfasst.

15. Formulierung gemäss Anspruch 1, ferner **dadurch gekennzeichnet, dass** die Formulierung im wesentlichen frei (wie hier definiert) von Metallionen ist.

16. Verfahren zur Herstellung einer Formulierung gemäss Anspruch 1, wobei das Verfahren das In-Verbindung-Bringen von Ibuprofen und Codein (oder einem pharmazeutisch akzeptablen Salz von einem oder beiden daraus) mit hydriertem pflanzlichem Öl umfasst.

## Revendications

1. Formulation pharmaceutique comprenant de l'ibuprofène ou un sel de celui-ci acceptable sur le plan pharmaceutique et de la codéine ou un sel de celle-ci acceptable sur le plan pharmaceutique en association avec un vecteur acceptable sur le plan pharmaceutique dans laquelle ledit vecteur comprend un agent lubrifiant, un désagrégeant et un diluant, **caractérisée en ce que** l'agent lubrifiant est substantiellement libre d'acide stéarique et d'ions stéarates, et est choisi parmi des huiles végétales hydrogénées.

2. Formulation selon la revendication 1 dans laquelle l'huile végétale hydrogénée est choisie parmi de l'huile de soja hydrogénée et de l'huile de ricin hydrogénée, et des mélanges de celles-ci.

3. Formulation selon la revendication 2 dans laquelle l'huile hydrogénée est choisie parmi des esters de glycérol ou des mélanges de ceux-ci.

4. Formulation selon la revendication 2 dans laquelle l'huile hydrogénée est choisie parmi les esters de glycérol C₁₆-C₂₀ et le tris 12-hydroxy stéarate de glycérol.

5. Formulation selon la revendication 1 dans laquelle le désagrégeant est choisi parmi un sel de carboxyméthylcellulose réticulée, un sel de glycolate d'amidon, une polyvinylpyrrolidone, et des mélanges de ceux-ci.

6. Formulation selon la revendication 5 dans laquelle le désagrégeant est choisi parmi la polyvinylpyrrolidone réticulée, le croscarmellose sodique et le glycolate d'amidon sodique ; et des mélanges de ceux-ci.

7. Formulation selon la revendication 5 dans laquelle le désagrégeant est, ou comprend, du glycolate d'amidon sodique.

8. Formulation selon la revendication 1 dans laquelle le diluant comprend du lactose, de la cellulose, ou des mélanges de ceux-ci, et de manière facultative de l'eau.

9. Formulation selon la revendication 8 dans laquelle le diluant est choisi parmi le Cellactose (nom commercial déposé), la cellulose microcristalline, et la carboxyméthylcellulose, et des mélanges de ceux-ci.

10. Formulation selon la revendication 8 dans laquelle le diluant comprend du lactose et de la cellulose dans un rapport de poids de substantiellement 3/1, et de manière facultative, de 3 à 5% m/m d'eau (en prenant pour base le poids du lactose et de la cellulose).

11. Formulation selon l'une quelconque des revendications précédentes dans laquelle ledit vecteur comprend en outre du dioxyde de silice colloïdale.

12. Formulation selon l'une quelconque des revendications précédentes comprenant en outre un principe actif choisi parmi la caféine, des décongestionnants, des antihistaminiques, des antitussifs ; et des mélanges de ceux-ci.

13. Formulation selon l'une quelconque des revendications précédentes comprenant 50-2400 mg d'ibuprofène et 10 à 30 mg de codéine.

14. Formulation selon l'une quelconque des revendications précédentes comprenant environ 200 mg d'ibuprofène et environ 13,57 mg de phosphate de codéine hémihydrate ; et, de manière facultative, environ 30 mg de caféine.

15. Formulation selon la revendication 1 **caractérisée en outre en ce que** la formulation est substantiellement libre (tel que défini dans ce document) d'ions métalliques.

16. Méthode pour la préparation d'une formulation selon la revendication 1, laquelle méthode comprend le fait d'associer l'ibuprofène et la codéine (ou un sel acceptable sur le plan pharmaceutique de l'un des deux) à l'huile végétale hydrogénée.
